**Europäisches Patentamt**

**European Patent Office**

**Office européen des brevets**

(11) Publication number: **0 231 037**

**A1**

# (12) EUROPEAN PATENT APPLICATION

(21) Application number: **87200012.0**

(22) Date of filing: 07.01.87

(51) Int. Cl.⁴: **A 61 B 6/03**
G 01 T 1/164

(30) Priority: **15.01.86 NL 8600067**

(43) Date of publication of application:
**05.08.87 Bulletin 87/32**

(84) Designated Contracting States: **DE FR GB NL**

(71) Applicant: **N.V. Philips' Gloeilampenfabrieken
Groenewoudseweg 1
NL-5621 BA Eindhoven (NL)**

(72) Inventor: **op de Beek, Johannes C. A.
c/o INT. OCTROOIBUREAU B.V. Prof. Holstlaan 6
NL-5656 AA Eindhoven (NL)**

(74) Representative: **Scheele, Edial François et al
INTERNATIONAAL OCTROOIBUREAU B.V. Prof.
Holstlaan 6
NL-5656 AA Eindhoven (NL)**

(54) **X-ray scanner with dual energy imaging.**

(57) An X-ray scanner comprising an array of detectors (16) and an X-ray tube (4) with elongated anode (26) or double focus can be operated in different modes:
- the unpaired mode by using all the detector signals separately
- the paired mode by combining the signals of two adjacent detectors, respectively
- the dual energy mode by selectively attributing different spectral filters (43, 45) to alternating detectors.

FIG.1

## Description

X-ray scanner with dual energy imaging.

The invention relates to an X-ray scanner with, mounted opposite each other in a rotatable holder, an X-ray source and an X-ray detection device with a row of interconnected detectors.

An X-ray scanner of this type is known from GB patent no. 1 527 728. In one of the embodiments described there, an X-ray tube and a detector array, preferably extending over the entire width of a fan-shaped X-ray beam to be emitted by the X-ray tube, are rotated around an object to be examined. For example, by using pulses from the X-ray source or detector signal integration at selected time intervals the absorption of a large number of irradiation paths through the object is measured. From the detector signals, an image of the object is then reconstructed from a disc irradiated by the fan-shaped beam.

In an X-ray scanner of this type, for example, by supplying two different high voltages in pulsed form to the X-ray tube and making use of spectrally sensitive detectors, what are termed dual energy measurements can be performed. A dual energy measuring method of this type is described in US patent no. 4,535,245. In this method of dual energy measurement there is the disadvantage that the object must be scanned twice in succession so that movement unsharpness can occur between the images of differing spectral energy. Extra measures must also be taken to ensure a rapid switchover of, for example, the high voltage supply of the X-ray tube and a correlated switchover of each of the detectors.

The object of the invention is to reduce the above disadvantages at least partially, and for this purpose according to the invention an X-ray scanner of the type mentioned in the preamble is characterised in that it is equipped for imaging in at least two different modes of detector signal processing and the row of detectors is divided into detector groups adapted for this which can be adjusted to differing spectral sensitivity.

Since in an X-ray scanner according to the invention the dual energy measurement in one of the imaging modes is carried out with a division of the detectors selected for this purpose, use can be made of the fact that in a mode with paired detectors signals from adjacent detectors can be combined in that mode during signal processing. For the dual energy measurement detectors from each of the combined sets are added to various detector groups which can be adjusted to a different spectral sensitivity. For imaging, these detectors are again read out individually and an image is formed from each of the groups. In particular, a set of detectors is formed each time by allocating from two adjacent detectors, one to a first group and one to a second group, the division resulting in a first detector group which comprises the even detectors in the arrangement of the row and a second detector group which comprises the uneven detectors from the row. An imaging mode of this type can be achieved particularly advantageously while maintaining resolution in an X-ray scanner equipped with an X-ray tube with a double focus, for example as described in US patent no. 4,010,370. If in the imaging mode in this the number of irradiation paths that can be measured by the rotating movement of the X-ray source and the detector device and by the moving focus is reduced by taking two detectors together each time, then one speaks of a beam addition 2 measuring method. The combined movement of the rotation and the moving focus make this measuring method very attractive and efficient in application. With the division into a detector group of even and uneven detectors, respectively, dual energy measurement can then be achieved. The signal processing here is completely analogous to the imaging mode for unpaired detectors, for there all the detectors are also read out. For example, in the case of the paired detector read-out half of a data flow is read twice and in the case of unpaired detectors, the whole detector current is read. To ensure good signal processing it may be advantageous to incorporate a temporary memory in the apparatus for the detector signals from each detector group.

In a preferred embodiment the detectors in each detector group are fitted with an input window with a specific radiation absorption adapted to a spectral energy path of the X-ray beam to be detected. For example, it is possible here to use materials with two adapted different K absorption sides located within the spectral path of the incident beam. In order to avoid any adverse influence when measuring without dual energy, the windows in a further embodiment can be moved between a position in the beam path and a position outside it. The windows here are, for example, formed by the teeth of a comb which can be folded away or pushed away.

On the basis of the drawing, some preferred embodiments according to the invention will be described in greater detail below. In the drawing:

Fig. 1 shows in very schematic form an X-ray scanner according to the invention and

Fig. 2 shows a part of a detector device for the scanner.

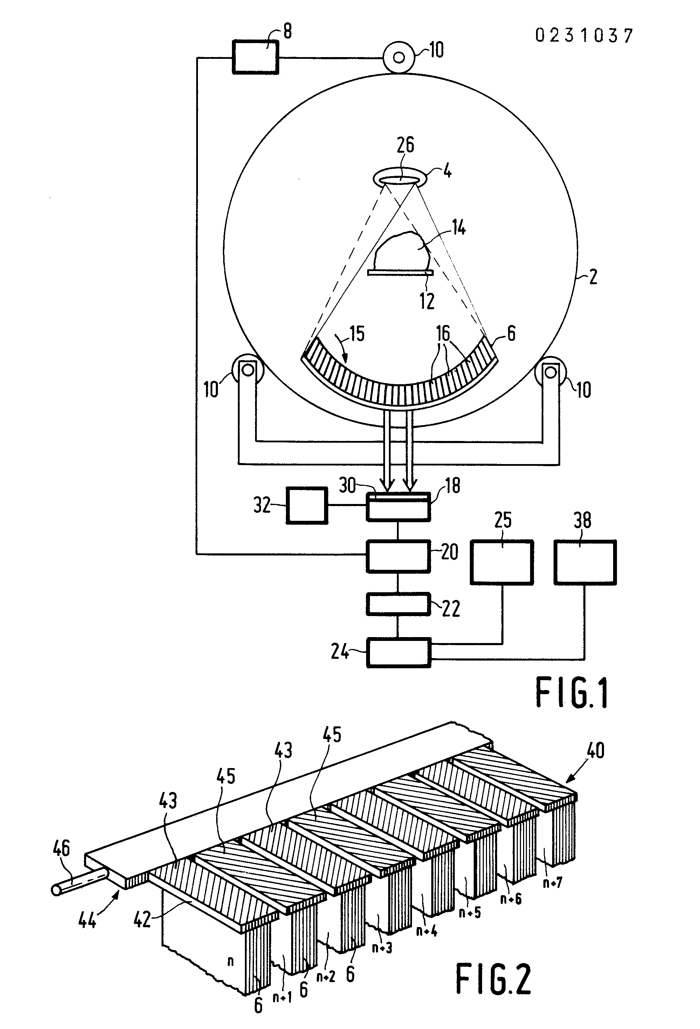

An X-ray scanner as indicated in Fig. 1 contains a rotatably positioned holder 2 in which an X-ray source 4 and a detection device 6 are incorporated. The holder is rotated by a drive motor 8 via a drive mechanism which is symbolised here by three drive rollers 10 around an object 14 lying on an object carrier 12. The detector device 5 contains a row of interconnected detectors 16, here arranged along an arc of circle, which can all be read out separately via a preamplifier 18 and an integrator 20. Detector signals obtained in this way are converted by means of an analog-digital convertor 22 and a computer 24 into an image signal which, for example, can be reproduced on a monitor 25. The X-ray source 4 here is an X-ray tube with an elongated anode 26 in the direction of rotation. An electron beam emitted by a cathode which is not indicated can be moved across the anode along a line located in the plane of rotation of the holder so that an X-ray focus travelling across the anode is formed.

An X-ray scanner of this type can be equipped for measuring in two different modes; for example, a first mode using all the detectors separately and thus with all the relevant beam paths which are formed during the combined scanning of an object by rotating the holder and moving the focus, here termed the unpaired measuring mode, and a second mode in which an image is formed by not using all the beam paths separately for the image reconstruction but through limiting this number by combining preferably two adjacent detectors each time, here termed the measuring mode with paired detectors.

A very practical division here is that, in the case of the mode with paired detectors, for each detector signal to be processed two successive detectors are combined and thus a beam addition 2 is used. Here it is then possible to use the same detector electronics for this mode. The number of data to be processed per time unit is the same. In the case of dual energy measurements, by alternately giving the detectors a different spectral sensitivity and reading these out alternately and individually, for which purpose a signal selector 30 is added, on-line absorption images can be formed for various radiation wavelengths. If the scanning speed is maintained for the dual energy measurement everything remains the same each time for half of the detection signals as far as data processing is concerned, which is extremely economical for the whole concept of the apparatus. A memory 32 can be added to the signal selector 30 for temporary storage of detector signals per detector group.

Some interconnected detectors 6 from the detection device 15 of the X-ray scanner are shown in perspective in Fig. 2. For each detector input surface 40 of the detectors there is an input window plate 42 which, for the detectors n, n+2, n+4, etc., is made of a material with a first radiation absorption and for the uneven detectors n+1, n+3, etc., is made of a material with a second radiation absorption. The window materials have been chosen in such a way that the irradiation which passes through them displays a substantial difference in spectral energy for the even detectors and the uneven detectors, respectively. This can, for example, be achieved by two materials with a K absorption side which, for example, are approximately 10 KeV apart from each other, but which both fall within the spectrum of the incident X-ray beam. In the drawing this difference is indicated by alternately hatching the even input windows 43 and uneven input windows 45 differently, which can therefore indicate a different material, different thickness, and so on. In order to achieve a more pronounced difference in wavelength a primary beam with, for example, two wavelength peaks can also be used, for example, from different radioactive sources, the window materials then being adapted to these peaks. For different materials for this purpose see GB patent no. 1 381 744.

By designing the window in the form of a comb 44 this can easily be moved in and out. For this purpose the comb can, for example, be mounted, for example, so that it can be tilted on a spindle 46. The accuracy required in the dimensioning of the detectors for scanners of this type makes it easy to adapt the comb to the detector array. This applies even more strongly to the scanner with a double focus in which beam addition is used in one of the imaging modes.

With the X-ray scanner described, on-line images can be formed which are generated by radiation with varying wavelength paths without any loss of spectral sensitivity. A second monitor 38 can be added for reproducing both images simultaneously. Linking up directly with imaging for high spectral energy a treatment planning system for possible irradiation therapy can be added. For high energy radiation the imaging is directly relevant for the required local dose distribution in irradiation.

## Claims

1. X-ray scanner with, mounted opposite each other in a rotatable holder, an X-ray source and an X-ray detection device with a row of interconnected detectors, characterised in that this is equipped for imaging in at least two different modes and the row of detectors is divided into detector groups adapted for this which can be adjusted to differing spectral sensitivity.

2. X-ray scanner according to claim 1, characterised in that beam addition 2 is used in one of the imaging modes and in that two detector groups are formed by successively adding detectors alternately to a different group.

3. X-ray scanner according to claim 1 or 2, characterised in that detector signals from at least one of the detector groups can be added to a memory for further processing.

4. X-ray scanner according to one of the above claims, characterised in that this is equipped for performing a rotational movement in a period of time which is adapted to processing a series of signals from both detector groups.

5. X-ray scanner according to one of the above claims, characterised in that each of the two series of measurement signals is reconstructed separately to form a sub-image.

6. X-ray scanner according to one of the above claims, characterised in that the X-ray source is an X-ray tube with a radiation focus which can be moved along a line in the rotational plane of the holder.

7. X-ray scanner according to one of the above claims, characterised in that each of the detector groups of an input window is fitted with a material with an absorption adapted to the spectral energy of the X-radiation to be detected.

8. X-ray scanner according to claim 7, characterised in that the window materials display a K absorption side, are located approximately 10 KeV apart, and both fall within the spectral path of the incident X-ray beam.

9. X-ray scanner according to claim 7 or 8, characterised in that the spectrally adapted input window for at least one of the detector groups can be moved between a position for the detectors in the radiation path and a position outside it.

10. X-ray scanner according to claim 7, 8 or 9, characterised in that the spectrally adapted input windows are formed by a comb with, for each detector of at least one of the groups, a tooth which covers the said detector.

11. X-ray scanner according to one of the above claims, characterised in that this is equipped for generating X-radiation with a spectral energy distribution with two radiation peaks, window material for the detection groups being adapted to these radiation peaks.

FIG.1

FIG.2

0231037

PHN 11601

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,X | US-A-4 535 245  (F.W.  ZONNEVELD et al.)<br>* Abstract; column 2, lines 5-16; column 2, line 53 - column 3, line 8; column 4, lines 34-59; figures 1,4 * | 1,3-5 | A 61 B    6/03<br>G 01 T    1/164 |
| | --- | | |
| X | US-A-4 149 081  (E.J.  SEPPI)<br>* Abstract; column 2, lines 23-29,36-49,57-68; column 3, lines 20-33; column 6, line 62 - column 8, line 36; figures 2a,2c * | 1-5 | |
| | --- | | |
| D,A | US-A-4 010 370  (C.A.G.  LE MAY)<br><br>* Abstract; column 3, lines 9-20,45-64; column 4, lines 47-68, column 9, lines 16-22; figures 1-3,5 * | 1,3,4, 6 | |
| | | | **TECHNICAL FIELDS SEARCHED (Int. Cl.4)** |
| | --- | | A 61 B<br>G 01 T |
| A | US-A-4 477 728  (T. RIFU)<br>* Abstract; column 1, lines 16-28; column 2, lines 36-61; column 3, lines 49-59; figures 1-6 * | 1,4,7 | |
| | --- | | |
| A | FR-A-2 490 479  (THOMSON-CSF)<br><br>* Page 5, line 20 - page 6, line 12; page 18, lines 9-32; figures 1,14-16 * | 1,3,4, 7,9 | |
| | ---          -/- | | |

The present search report has been drawn up for all claims

| Place of search | Date of completion of the search | Examiner |
|---|---|---|
| THE  HAGUE | 21-04-1987 | RIEB K.D. |

## DOCUMENTS CONSIDERED TO BE RELEVANT

Page 2

| Category | Citation of document with indication, where appropriate, of relevant passages | Relevant to claim | CLASSIFICATION OF THE APPLICATION (Int. Cl.4) |
|---|---|---|---|
| A | FR-A-2 353 865 (EMI LTD) <br><br> * Page 3, line 10 - page 4, line 28; page 9, line 25 - page 10, line 23; figures 1,5 * | 1,2,4, 6,10 | |
| A | US-A-3 854 049 (C.A. MISTRETTA et al.) <br> * Abstract; column 3, lines 16-39; column 3, line 61 - column 4, line 14; column 5, lines 30-65; figures 1-4 * | 5,7-9, 11 | |
| | | | TECHNICAL FIELDS SEARCHED (Int. Cl.4) |

The present search report has been drawn up for all claims

| Place of search | Date of completion of the search | Examiner |
|---|---|---|
| THE HAGUE | 21-04-1987 | RIEB K.D. |